(19) Europäisches Patentamt
European Patent Office
Office européen des brevets

(11) Veröffentlichungsnummer: 0 110 429
B1

# (12) EUROPÄISCHE PATENTSCHRIFT

(45) Veröffentlichungstag der Patentschrifft: 17.01.90

(51) Int. Cl.[4]: C 08 G 73/04

(21) Anmeldenummer: 83112248.6

(22) Anmeldetag: 06.12.83

## (54) Verzögerer für die Polymerisation von Aziridinverbindungen.

(30) Priorität: 06.12.82 DE 3245052

(43) Veröffentlichungstag der Anmeldung: 13.06.84 Patentblatt 84/24

(45) Bekanntmachung des Hinweises auf die Patenterteilung: 17.01.90 Patentblatt 90/03

(84) Bennante Vertragsstaaten: AT BE CH DE FR GB IT LI NL SE

(56) Entgegenhaltungen:
DE-A-2 515 593
US-A-3 453 242
US-A-4 093 555
US-A-4 167 618

(73) Patentinhaber: ESPE Stiftung & Co Produktions- und Vertriebs KG
Am Griesberg 2
D-8031 Seefeld (DE)

(72) Erfinder: Jochum, Peter, Dr.
Pointweg 5
D-8031 Seefeld 2 (DE)
Erfinder: Hübner, Heijo, Dr.
Moosbichlweg 16
D-8031 Wörthsee/Steinebach (DE)
Erfinder: Gasser, Oswald, Dr.
Hartstrasse 13
D-8031 Seefeld (DE)

(74) Vertreter: Abitz, Walter, Dr.-Ing.
Abitz, Morf, Gritschneder, Freiherr von Wittgenstein
Postfach 86 01 09
D-8000 München 86 (DE)

EP 0 110 429 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Zur Herstellung von präzisen Abdrücken, besonders in der Zahnheilkunde, von Arbeitsmodellen, insbesondere für die Zahntechnik, und von provisorischen Zahnersatzteilen werden aziridinhaltige Substanzen polymerisiert, wie sie z. B. in den US-PSen 3 453 242 und 4 093 555 beschrieben sind. Wie in den genannten Vorveröffentlichungen beschrieben, werden die aziridinhaltigen Verbindungen zusammen mit Füllstoffen, Farbstoffen und weiteren Hilfsstoffen verwendet.

Für einige Anwendungszwecke, besonders in der Zahnmedizin und Zahntechnik, ist es von Bedeutung, dass die Verarbeitungszeit der initiierten Mischung und das Ende der Polymerisationsreaktion in relativ engen Grenzen festgelegt werden kann. Dies gilt z. B. bei der Herstellung eines Kieferabdrucks: Die Verarbeitungszeit der initiierten Mischung muss ausreichend sein, um das Material auf einen Abdrucklöffel und anschliessend in den Mund des Patienten zu bringen. Anschliessend soll die Polymerisation möglichst rasch beendet sein, damit der Abdruck nach kurzer Zeit ohne Dimensionsänderung entnommen werden kann und die zeitliche Belastung für Zahnarzt und Patient erträglich bleibt. Ähnliches gilt bei der intraoralen Herstellung eines provisorischen Zahnersatzteils, aber auch bei der Herstellung eines Kiefermodells durch den Zahntechniker.

Aus der US-PS 4 167 618 ist es bekannt, Sulfoniumsalze, die in ß-Stellung zum zentralen Schwefelatom eine elektronenanziehende Gruppe sowie ein nichtnukleophiles Anion enthalten, als Polymerisationsstarter für diese Aziridinverbindungen zu verwenden.

Verarbeitungszeit (Topfzeit) und Abbindezeit (Erhärtungszeit) der Mischungen lassen sich bei Verwendung von Sulfoniumsalzen als Polymerisationsinitiatoren in relativ weiten Grenzen durch die chemische Konstitution des Sulfoniummoleküls beeinflussen.

Wird ein wenig aktiver Sulfoniumstarter verwendet, so steht zwar genügend Verarbeitungszeit zur Verfügung, die Erhärtung erfolgt jedoch so langsam, dass dies für die Anwendung, besonders im zahnärztlichen Bereich bei der Abdrucknahme, zu einer unerträglichen Verzögerung der Erhärtung der gummielastischen Abdruckmasse führt. Ein aktives Sulfoniumsalz bewirkt zwar ein rasches Ende der Gummibildung, so dass z. B. ein Abdruck nach kurzer Zeit dem Mund entnommen werden kann, ohne dass Verformungen des Abdrucks auftreten. Dann ist jedoch die Verarbeitungszeit so kurz, so dass besonders in der warmen Jahreszeit die Gefahr besteht, dass der Abdruck bereits in der Reaktionsphase in den Mund eingebracht wird und die Masse schon ein gewisses Rückstellvermögen zeigt, oder es zu Verdrängungen im Zahnfleischbereich kommt.

Bei der Auswahl des Sulfoniumsalzes sind neben dem gewünschten zeitlichen Ablauf der Polymerisation weitere Forderungen zu beachten: Verfügbarkeit der Ausgangskomponenten zur Erzielung der gewünschten Substitution, Möglichkeiten einer geeigneten Reaktionsführung, thermische und hydrolytische Stabilität des Produkts, sowie die Löslichkeit des Sulfoniumsalzes in der ausgewählten Aziridinverbindung. Oft ist es nicht möglich, ein Sulfoniumsalz zu finden, das den gewünschten Polymerisationsverlauf ohne grössere Abweichungen bewirkt. Nachteilig wirkt sich dabei weiterhin aus, dass der Polymerisationsverlauf nur in geringem Umfang durch eine Mengenvariation des Sulfoniumsalzinitiators beeinflusst werden kann.

Es ist bekannt, dass Amine die Polymerisation von Aziridinverbindungen verzögern können. Die üblichen organischen Amine (z. B. Tributylamin, Benzylamin, Triethanolamin) zeigen jedoch entweder keine Wirkung oder verzögern die Erhärtung so stark, dass ihr Einsatz nicht möglich ist.

Aufgabe der Erfindung ist demnach die Bereitstellung von Verbindungen, die die Gelierung der mit Sulfoniumsalzen initiierten Polymerisation von Aziridinverbindungen verzögern, ohne die Erhärtungszeit wesentlich zu verlängern.

Es wurde gefunden, dass Imidazole die Verarbeitungszeit von mit Sulfonium-Initiatoren vermischten Aziridinverbindungen verlängern, ohne die Erhärtung nennenswert zu beeinflussen.

Gegenstand der Erfindung ist demnach eine mit Sulfoniumsalzen polymerisierbare Masse auf Basis mindestens einer Aziridinverbindung, wobei diese Masse mindestens eine Imidazolverbindung in gelöster Form enthält. Die Erfindung betrifft ausserdem die Verwendung von in Aziridinverbindungen auflösbaren Imidazolen als Verzögerer der Gelierung der mit Sulfoniumsalzen initiierten Polymerisation von Aziridinverbindungen.

Die erzielbare Verzögerung der Erhärtung beträgt je nach Konzentration des Imidazolderivats ca. 15 Sekunden bis einige Minuten. Der erfindungsgemässe Zusatz von Imidazolen zu den aziridinhaltigen, polymerisierbaren Massen ermöglicht die Verwendung eines sehr reaktiven Sulfoniumstarters (vorzugsweise mit einem Nitrilsubstituenten in ß-Stellung zum zentralen Schwefelatom), und gewährleistet so gleichzeitig eine ausreichende Verarbeitungszeit und eine rasche Erhärtung der Masse.

Prinzipiell sind Imidazol selbst und alle 1-substituierten Imidazolderivate für den erfindungsgemässen Einsatz geeignet, sofern sie in der (den) jeweils eingesetzten Aziridinverbindung(en) auflösbar sind. In den eingesetzten Aziridinverbindungen schwerer lösliche Imidazole können durch Zugabe von Weichmachern, wie Estern der Phthalsäure, als Lösungsvermittler in Lösung gebracht werden. Bevorzugt werden 1-substituierte Imidazole eingesetzt.

Beispielsweise können die erfindungsgemäss verwendeten Imidazole die allgemeine Formel

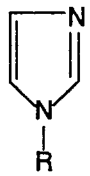

aufweisen, worin R bedeutet

(a) $C_1$-$C_{18}$-Alkyl
(b) substituiertes $C_1$-$C_{18}$-Alkyl
(c) $C_3$-$C_{12}$-Cycloalkyl
(d) substituiertes $C_3$-$C_{12}$-Cycloalkyl.
(e) $C_2$-$C_{18}$-Alkenyl
(f) substituiertes $C_2$-$C_{18}$-Alkenyl
(g) substituiertes Phenyl
(h) H

Der Alkylrest in (a) und (b) hat bevorzugt 1 bis 12 C-Atome, der Cycloalkylrest in (c) und (d) 3 bis 6, besonders 5 oder 6 C-Atome, und der Alkenylrest in (e) und (f) 2 bis 12 C-Atome, besonders Vinyl und Allyl.

Als Substituenten für die Reste (b), (d), (f) und (g) kommen in Betracht Ester-Reste, z. B. $C_1$-$C_{18}$-Alkoxycarbonylreste; Amidocarbonylreste, z. B. $C_1$-$C_{18}$-Alkylamidocarbonylreste oder Aralkylamidocarbonylreste, wie Benzylamidocarbonyl; Acylamidoreste, z. B. $C_2$-$C_{18}$Alkanoylamido- oder Benzoylamidoreste; Acyloxyreste, z. B. $C_2$-$C_{18}$-Alkanoyloxy- oder Benzoyloxyreste; und Ether-Reste, vorzugsweise mit 1 bis 18 C-Atomen, z. B. $C_1$-$C_{18}$-Alkoxyreste. Auch - gegebenenfalls substituierte-Phenylreste und 1-Imidazolylreste sind als Substituenten für die Reste (b), (d), (f) und (g) geeignet.

Beispiele für geeignete Imidazolverbindungen sind 1-Methyl-imidazol, 1-(n-Butyl)-imidazol, 1-Decyl-imidazol, 1-Lauryl-imidazol, 1,-Bis-(1-imidazolyl)-$C_1$-$C_{10}$-alkane, wie 1,2-Bis-(1-imidazolyl)-ethan und 1,10-Bis-(1-imidazolyl)-decan, 11-(1-Imidazolyl)-undecansäurebenzylamid, 1-Cyclohexyl-imidazol, 1-Benzyl-imidazol, 1-(2-Ethoxyethyl)-imidazol, 1-(4-Methoxy-phenyl)-imidazol und 1-[3-(2-ethylhexanoyl)-amidopropyl]-imidazol.

Die Imidazole werden im allgemeinen in einer Menge von 0,01 bis 3, vorzugsweise 0,05 bis 2 %, bezogen auf das Gewicht der Aziridinverbindungen, eingesetzt.

Die Herstellung der erfindungsgemäss verwendeten Imidazole erfolgt nach bekannten Verfahren, die z. B. in J. Chem. Soc., *1963*, 2197 und Helv. Chim. Acta, *42*, 1845 (1959) beschrieben sind.

Als Aziridinverbindungen können die eingangs erwähnten Verbindungen der US-PSen 3 453 242 und 4 093 555 Verwendung finden, z. B. Polyether mit Aziridino-Endgruppen und Bisphenol-A-Derivate mit Aziridino-Endgruppen. Als Sulfoniuminitiatoren sind diejenigen der US-PS 4 167 618 geeignet, wobei die Sulfoniumsalze, die in ß-Stellung zum S-Atom Nitril- oder Estergruppen enthalten, bevorzugt sind. Deren Mengen betragen 1 bis 8 % bei Aziridinverbindungen, wie sie in der US-PS 4 093 555 beschrieben sind, bei anderen Aziridinverbindungen 2 bis 20 %, bezogen auf das Gewicht der Aziridinverbindungen.

Die mit den erfindungsgemäss verwendeten Imidazolen vermischten Aziridinverbindungen finden Verwendung zur Herstellung von präzisen Abdrücken, von Arbeitsmodellen und vor allem in dentalen Abdruckmassen auf der Basis von Polyethern mit Aziridinoendgruppen; die Herstellung derartiger Aziridinopolyether ist in der US-PS 3 453 242 beschrieben. Zur Herstellung der Abdruckmasse werden diesem Aziridino-Polyether Füllstoffe, Weichmacher Farbstoffe, Geschmackskorrigentien und andere übliche Hilfsstoffe zugesetzt.

Strukturell verwandte Verbindungen, nämlich N-substituierte Pyrazole und Triazole, weisen die erfindungsgemäss erwünschte Wirkung (Verzögerung des Beginns der Gelierung bei Zusatz des Sulfoniumsalzes zur Aziridinverbindung) nicht auf.

Die in den Beispielen genannten Zeiten sind jeweils ab Beginn der Anmischung der Aziridinokomponente mit dem Sulfoniumstarter gerechnet.

**Beispiel 1**

1,0 g eines Polyethers mit Aziridinoendgruppen, der ein durchschnittliches Mol-Gewicht von ca. 6500 besitzt und dessen Herstellung in der US-PS 3 453 242 (Beispiel 13) beschrieben ist, wird mit 4 mg 1-Methyl-imidazol vermischt. Dieses Gemisch wird dann mit 0,08 g ß-(S-Lauryl-S-ethylsulfonium)butyronitrilfluoroborat homogen vermischt. Nach 3 Minuten tritt Gelierung ein, nach 5,5 Minuten ist eine staubtrockene, gummielastische Masse entstanden.

**Vergleichsbeispiel 1**

Das Verfahren des Beispiels 1 wird ohne Zusatz des Imidazols wiederholt. Bei einer Raumtemperatur von ca. 23°C tritt nach 1 Minute 50 Sekunden Gelierung ein, nach 4 Minuten 30 Sekunden ist eine staubtrockene, gummielastische Masse entstanden.

**Beispiel 2**

1,0 g des im Beispiel 1 genannten Aziridino-polyethers wird mit 8 mg 1-Lauryl-imidazol vermischt. Anschliessend werden 0,16 g eines 1 : 1 Gemisches aus Diethylhexylphthalat und dem im Beispiel 1 genannten Sulfoniumsalz homogen eingearbeitet. Die Masse beginnt nach 3 Minuten zu gelieren und hat sich nach 5 Minuten 30 Sekunden zu einer staubtrockenen, gummielastischen Masse verfestigt.

**Beispiel 3**

Zur Herstellung einer Abdruckmase für zahnärztliche Zwecke werden 800 g der im Beispiel 1 genannten bifunktionellen Aziridinverbindung mit 150 g feinem Kieselgur verknetet; ausserdem werden 3,2 g 1-Methyl-imidazol zugesetzt. 30 g dieser Paste werden mit 4,8 g eines 1 : 1 Gemisches aus Diethylhexylphthalat und dem im Beispiel 1 genannten Sulfoniumsalz vermischt. Dem Zahnarzt stehen 3 Minuten zur Verfügung, um das Abdruckmaterial mit Hilfe eines geeigneten Löffels in den Mund des Patienten einzubringen. Nach 5 Minuten 30 Sekunden ist die Gummibildung soweit abgeschlossen, dass der Abdruck ohne Verformungsgefahr dem Mund des Patienten entnommen werden kann.

**Beispiel 4**

In 1,0 g des in Beispiel 1 genannten Aziridinopolyethers werden 0,5 mg 1-Methyl-imidazol gelöst. Anschliessend werden 0,2 g eines 1 : 1-Gemisches aus Acetyl-tributyl-zitrat und 2-Ethylhexyl-oxycarbonylmethyl-ethylsulfonium-propion säure-tetradecylester-fluoroborat homogen eingearbeitet. Die Gelierung tritt nach 3 Minuten ein, nach 9 Minuten ist eine staubtrockene, gummielastische Masse entstanden.

**Vergleichsbeispiel 2**

Das Verfahren des Beispiels 4 wird ohne Zusatz des Imidazols wiederholt. Die Gelierung tritt jetzt nach 2 Minuten 40 Sekunden ein, nach 8 Minuten 30 Sekunden ist wiederum eine staubtrockene, gummielastische Masse entstanden.

**Patentansprüche**

1. Mit Sulfoniumsalzen polymerisierbare Masse auf Basis mindestens einer Aziridinverbindung, enthaltend mindestens eine Imidazolverbindung in gelöster Form.

2. Verwendung von in Aziridinverbindungen auflösbaren Imidazolen als Verzögerer der Gelierung der mit Sulfoniumsalzen initiierten Polymerisation von Aziridinverbindungen.

**Revendications**

1. Composition polymérisable par des sels de sulfonium, à base d'au moins un composé de l'aziridine, contenant au moins un composé de l'imidazole sous forme dissoute.

2. Utilisation d'imidazoles solubles dans les composés de l'aziridine en tant que retardateur de la gélification de la polymérisation, amorcée par des sels de sulfonium, de composés de l'aziridine.

**Claims**

1. Composition which is polymerizable with sulfonium salts, based on at least one aziridine compound, containing at least one imidazole compound in dissolved form.

2. Use of imidazoles soluble in aziridine compounds as retarders for the gelling of the polymerization of aziridine compounds initiated with sulfonium salts.